# EUROPEAN PATENT SPECIFICATION

(11) **EP 0 910 287 B1**
(45) Date of publication and mention of the grant of the patent: **19.12.2007**
(21) Application number: 96916751.9
(22) Date of filing: 04.06.1996
(51) Int. Cl.: A61B 17/00

(54) **KNOTLESS SUTURE ANCHOR ASSEMBLY**
KNOTENLOSER NÄHFADENANKERAUFBAU
ENSEMBLE D'ANCRAGE DE SUTURE SANS NOEUD

(30) Priority: 06.06.1995 US 471508; 01.11.1995 US 551648
(43) Date of publication of application: 28.04.1999
(73) Proprietor: Thal, Raymond, McLean, VA 22102 (US)
(72) Inventor: Thal, Raymond, McLean, VA 22102 (US)
(74) Representative: Basfeld, Rainer
(86) International application number: PCT/US1996/007961
(87) International publication number: WO 1996/039082

(56) References cited:
- EP-A- 0 589 306
- EP-A- 0 632 999
- US-A- 5 074 874
- US-A- 5 141 520
- US-A- 5 152 790
- US-A- 5 259 846
- US-A- 5 268 001
- US-A- 5 370 661
- US-A- 5 500 000

## Description

### 1. Field of the Invention

The present invention relates to devices or assemblies used in tissue repair. More particularly, the assembly enables the attachment together or repair of portions of biological tissue (i.e., tendons or ligaments) onto a bone surface.

### 2. Description of the Background Art

Soft tissues, such as tendons and ligaments, generally are attached to bone by small collagenous fibers. These connections are strong but permit the tendons and ligaments to be flexible. When a tissue is torn away from the bone and requires repair, a surgeon is often required to repair the detached soft tissue with sutures which are passed through bone tunnels and tied. A number of devices have been developed for securing a ligament or tendon to a bone mass. These devices can be used in place of bone tunnelling techniques. These attachment devices are usually inserted through extensive surgical incisions and, in some circumstances, by arthroscopic surgical techniques. The use of bone tunnels for repair can be difficult and generally require large open incisions. Recently, through the advent of endoscopic surgery, where the surgeon looks into a joint cavity with a telescope, there has been a trend to repair soft tissues back to bone through small incisions called portals. The unique knotless suture anchor assemblies described herein facilitate this difficult and precise procedure.

A variety of devices are available for attaching objects to bone, such as screws, staples, cement, suture anchors, and sutures alone. These devices have been used to attach soft tissue, such as ligaments, tendons, muscles, as well as objects such as protheses, to bone. A suture anchor is a device which utilizes small anchors with suture materials attached thereto. A device, such as a screw, is inserted into the bone mass and anchored in place. After insertion of the anchor, the attached suture is passed through the tissue to be repaired. The tying of a knot in the suture is then require to secure the tissue to the bone. The process of passing the anchored suture through the soft tissue and tying a knot is time consuming and difficult to undertake in the tight space encountered during endoscopic surgery and sometimes even in conventional open surgery.

One example of a suture anchor assembly is disclosed in U.S. Patent No. 5, 370, 662, wherein an anchor assembly includes a pre-threaded suture positioned at its posterior. First the anchor is inserted into the bone mass. The attached suture is then passed through the tissue for reattachment. The surgeon is required to tie a knot with the suture to complete the surgical process. Some suture anchors can be passed through the soft tissue first and then into the bone. Most suture anchors need to be inserted into the bone first. Only after this has been accomplished can the sutures be passed through the soft tissue. Alternatives to this procedure include non-suture soft tissue anchor systems. A few of these systems, such as those disclosed in U.S. Patent Nos. 5,013,316 and 4,532,926, can be used arthroscopically but fixation with these devices may not be as secure as that achieved with sutures. Only a few points of fixation are possible with the non-suture type anchor since the device is relatively large. Therefore suture devices are more favorable. This type of non-suture staple device is disadvantageous in that it has been known to crack the bone during deployment, or accidentally transect the object being attached to the bone. In addition, the device itself has been known to crack or break during or after deployment.

U.S. Patent Nos. 5,037,422; 5,224,946; and 5,236,445 all disclose bone anchor configurations for attaching sutures within openings formed in bones during joint reconstructive surgery and endoscopic surgical procedures. With all these intricate procedures, the suture itself must be inserted through a tissue mass and tied with a surgical knot to repair the soft tissue to bone.

A primary object of the present invention is to provide a suture anchor assembly which is easy to use and install.

Another object of the present invention is to provide a suture anchor assembly which allows for secure attachment of soft tissue to bone without the use or requirement of tying a knot.

Still another object of the present invention is to provide a suture anchor assembly which is compact and allows a surgeon to easily guide the tissue into a bone anchoring sleeve to enhance the security of the repair.

Still another object of the present invention is to provide an anchor assembly which allows for passage through soft tissue in a singular fashion without the need for additional instrumentation for passing the suture separately through the soft tissue to be repaired.

### SUMMARY OF THE INVENTION

In accordance with the above objects, the present invention is a knotless suture anchor assembly according to claim 1 for attachment or reattachment of biological soft tissue to bone as set out in the according claims. The unique knotless suture anchor assembly includes a hollow anchoring sleeve which is installed into a bone mass. The anchoring sleeve can have a closed pointed drill end or be totally cylindrical in shape. The hollow anchoring sleeve can be ribbed or threaded on its exterior for secure attachment to the bone or embody varying types of anchor configurations to facilitate a strong bond with the bone mass A number of prior patents, see e.g. EP-A-632 999, disclose configurations for the exterior of a bone anchor which are within the contemplation of the invention for use as the anchoring means for the exterior of the hollow anchoring sleeve.

U.S. Patent Nos. 4,007,743; 4,632,101; 4,721,103; 4,870,957; 4,898,156; 4,946,468; 5,084,050; 5,102,421; 5,141,520; 5,192,303; and 5,207,679, all illustrate varying exterior structures which may embody the anchoring sleeve portion of the invention. These patents disclose various means and mechanisms for anchoring a device to a bone mass thus preventing pull-out of the sleeve after insertion into bone.

Further, the hollow anchoring sleeve can contain a collar on the rear portion or rear side of the hollow anchoring sleeve to control the depth of sleeve insertion into the bone and prevent excessive insertion depth.

A second component of the knotless suture anchor assembly is the spike or plug member which has on its first end a configuration which allows for easy puncturing of a soft tissue and on its second or other end a means for attachment of a suture material. The first end can be pointed or frustoconical in shape. The spike or plug can be ribbed, beaded, threaded or expandable on its exterior surface for secure mating with the interior wall section of the hollow anchoring sleeve. The suture material which is attached to the rear end of the spike or plug member has at least one loop portion positioned at its other end. The loop portion can be the same suture material as the suture which is attached to the end of the spike or plug. Alternatively, the loop portion can be produced of a different material, other than suture, with different characteristics (i.e. - molded or metal) and can be attached to the spike or plug by a length of suture. In the alternative, the suture can be attached in one full loop on the rear end of the spike or plug member.

The spike or plug member, suture and loop portion can be all produced of the same material (i.e., molded). This would obviate need for the second end of the spike or plug member to have means for attachment of the suture thereto.

The spike or plug member is inserted during an open or endoscopic procedure, or the like, through the soft tissue and its piercing or pointed end is then threaded through the loop portion formed in the suture material prior to its ultimate insertion into the anchoring sleeve to facilitate a secure mating. Once the spike or plug member is threaded through the tissue and then through the loop portion and is inserted into the hollow anchoring sleeve, it is then securely attached through pressure by the surgeon into the sleeve. This attachment of the spike member to the hollow anchoring sleeve can be accomplished in one step or in a number of depth control steps (i.e., ratchets) to fine tune the tightness of the repair. This ratchet effect can be accomplished by a series of beads, ribs, thickening or the like on the exterior of the spike component. These would mate with the interior of the anchor sleeve. This allows for the tissue to be tightly attached to the bone mass. The unique device obviates the need for the surgeon to tie a knot with the suture material for reattachment of tissue to bone. Endoscopic procedures and some open surgical procedures are extremely difficult and must be completed in a very tight space. Obviation of the need of tying a knot is extremely beneficial and innovative.

Further, the unique knotless anchoring sleeve may be formed in one complete assembly. The hollow anchoring sleeve has one end for insertion into the bone mass and a suture material attached at its other end. The other end of the suture is attached to the rear end of the spike or plug member. This spike or plug member can then be inserted directly through the tissue mass and into the hollow anchoring sleeve for attachment of soft tissue to bone. The length of the suture material is adjustable based upon the requirements of the surgical procedure. The procedure is a process wherein first the spike or plug member which may be ribbed, beaded, threaded or expandable is inserted through the soft tissue and then directly into the hollow anchoring sleeve. The hollow anchoring sleeve is then inserted into the bone mass. The soft tissue is thus secured to the bone mass. Tension is then adjusted by the depth of the anchor insertion and/or the ribbed, beaded, threaded or expandable mechanism for mating the spike member into the hollow anchoring sleeve.

As previously described, the suture and loop portion can vary in shape and be produced of the same or different materials.

Numerous other features of various embodiments of the knotless suture anchor assembly will be apparent from the following detailed description and the accompanying drawings.

### BRIEF DESCRIPTION OF THE DRAWINGS

**FIGS. 1a, 1b, 1c and 1d** are perspective views of a hollow anchoring sleeve suitable for use with the present invention;
**FIG. 2** is a perspective view of a spike member with suture element suitable for use with the present invention;
**FIG. 3** is a perspective view of an alternate embodiment of a spike member with suture element suitable for use with the present invention;
**FIG. 4** illustrates usage an embodiment in conjunction with a bone mass and tissue during surgical reattachment;
**FIG. 5** illustrates an embodiment in place after attachment of tissue to bone mass has been completed;
**FIG. 6** illustrates a knotless suture anchor assembly;
**FIG. 7** illustrates the procedure for attachment of tissue to bone mass for the embodiment as outlined in FIG. 6;
**FIG. 8** illustrates the embodiment of FIG. 6 showing tissue attached to bone;
**FIG. 9** illustrates the spike components of a knotless suture anchor assembly suitable for use with the present invention;
**FIG. 10** is a partial exploded view of the spike components and hollow anchoring sleeves of the present invention inserted through soft tissue and mated together for attachment of tissue to bone mass;
**FIG. 11** is an illustration of the embodiment of FIGS. 9 and 10 wherein tissue is attached to a bone mass in a secure manner;
**FIG. 12** illustrates an alternate embodiment suitable for use with the present invention;
**FIG. 13** illustrates the embodiment of FIG. 12 in conjunction with a bone mass and tissue during surgical reattachment;
**FIG. 14** illustrates an alternate embodiment suitable for use with the present invention;
**FIG. 15** illustrates the embodiment of FIG. 14 in conjunction with a bone mass and tissue during surgical reattachment;

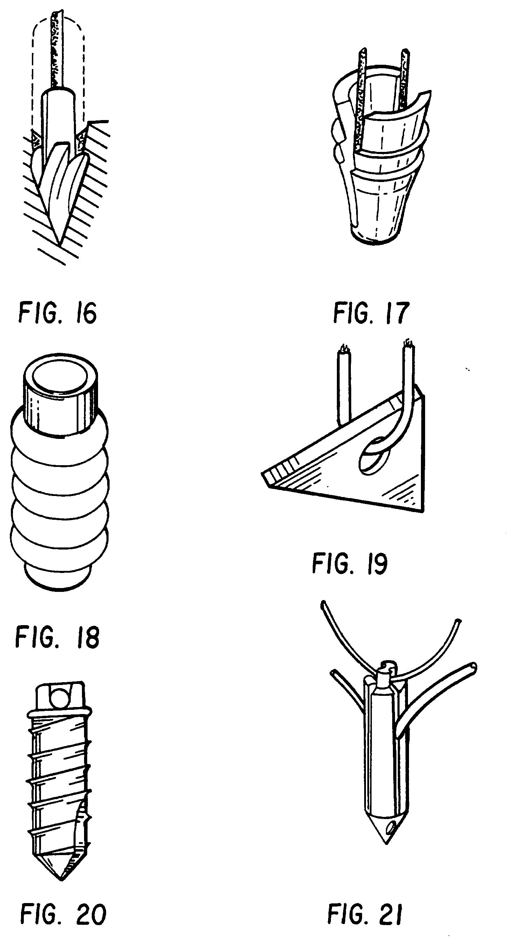

**FIG. 16** is an enlarged perspective view of a harpoon anchor;
**FIG. 17** is an enlarged perspective view of an umbrella-like anchor;
**FIG. 18** is an enlarged perspective view of a ribbed anchor;
**FIG. 19** is an enlarged perspective view of a wedge-like anchor;
**FIG. 20** is an enlarged perspective view of a threaded anchor;
**FIG. 21** is an enlarged perspective view of a pronged anchor;
**FIGS. 22a and 22b** are perspective views of a spike member with suture element and stop means made suitable for use with the present invention;
**FIGS. 23a and 23b** are perspective views of an alternate embodiment of a spike member with suture element suitable for use with the present invention;
**FIGS. 24a and 24b** are perspective views of an alternate embodiment of a spike member with suture element suitable for use with the present invention;
**FIGS. 25a and 25b** are perspective views of an alternate embodiment of a spike member with suture element suitable for use with the present invention;
**FIGS. 26a and 26b** are perspective views of an alternate embodiment of a spike member with suture element suitable for use with the present invention;
**FIG. 27** illustrates the procedure for attachment of tissue to bone mass for the embodiment as outlined in FIG. 22b;
**FIG. 28** illustrates the procedure for attachment of tissue to bone mass for the embodiment as outlined in FIG. 22a; and
**FIG. 29** illustrates one procedure for attachment of tissue to bone mass for the embodiment as outlined in FIG. 23b.

### DETAILED DESCRIPTION OF THE PRESENT INVENTION

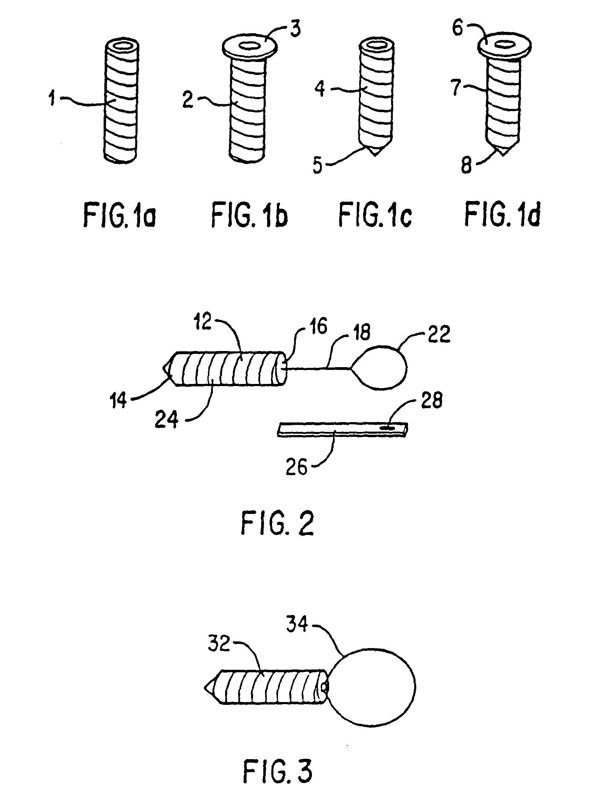

Referring now to **FIG. 1,** the knotless suture anchor assembly suitable for use with the present invention which contains as one integral component a hollow anchoring sleeve for installation and attachment to a bone mass. The hollow anchoring sleeve **1,** as shown in **FIG. 1a,** is cylindrical in shape and possesses ribs or threads on its exterior. The device can also contain or be configured with prongs, umbrella spokes, have threads, be expandable, or have wedges, on its exterior, for secure attachment with the bone mass. These exterior attachment features are known to the industry.

**FIG. 1b** illustrates an alternate embodiment of the hollow anchoring sleeve **2** having a collar **3** to control depth of bone penetration. The collar prevents the sleeve from being forced too deep into the bone mass when the spike or plug member is inserted.

**FIG. 1c** illustrates an alternate embodiment of the hollow anchoring sleeve **4** wherein the sleeve has a pointed closed end **5** for ease of penetration into a bone mass.

**FIG. 1d** illustrates a hollow anchoring sleeve **7** with a collar **6** and a closed pointed end **8** as an alternate construction.

As pointed out in the Summary of the Invention, the hollow anchoring sleeve may also be shaped or configured with any means to secure said structure to a bone mass. The hollow anchoring sleeve may include a threaded exterior as disclosed in U.S. Patent No. 5,370,662. Further, the device may be expandable as disclosed in U.S. Patent No. 5,084,050. A configuration such as disclosed in U.S. Patent Nos. 5,037,422; 5,224,946; and 5,236,445 is also suitable for use with the invention. Harpoon configurations such as disclosed in U.S. Patent Nos. 5,141,520 and 5,102,421 are also contemplated for the hollow anchoring sleeve.

It is also within the contemplation of the present invention to configure the anchoring sleeve in a harpoon-type fashion such as disclosed by U.S. Patent Nos. 4,632,101 and 4,721,103 for secure anchoring within the bone mass. U.S. Patent Nos. 4,898,156; 5,207,679; 4,946,468; and 5,192,303 disclose anchoring mechanisms which can be utilized for the hollow sleeve member for installation within a bone mass. It is also within the contemplation of the present invention to configure this dowel-like hollow sleeve in any fashion to securely attach same to a bone mass.

The interior surface of the hollow anchoring sleeve is ribbed, beaded, threaded, expandable or smooth for secure engagement with said exterior surface of said spike member.

**FIG. 2** shows a perspective view of the spike plug member with suture element to be used with the present invention. Spike or plug member **12** is preferably cylindrical in shape with a sharp first end **14** and a second end **16** wherein the suture element **18** is attached. The suture element **18** has at its distal end a loop or ring **22.** The loop or ring portion **22** can be constructed of the same suture material as suture element **18** or it can be a separate molded material attached to the suture element **18** and loop portion **22** may be one molded component and attached to spike member **12** at end **16** or they may be all (spike, suture element and loop portion) one entire molded component. Further, the suture element **18,** alone, can be made from any type suture material which has been approved for surgical procedures for attachment of tissue to bone. The spike or plug member can form any shape so long as it mates with the hollow cylindrical sleeve as described above. The exterior of the spike or plug member **12** may be ribbed or threaded **24** as depicted in **FIG. 2** or may be beaded or expandable to allow for a secure tight fit with the inner hollow cylinder of the anchoring sleeve. Once inserted into a hollow anchoring sleeve, the exterior surface of the spike or plug member **12** engages the inner surface of the sleeve and can be ratcheted down to produce the desired tight fit. This ratcheting effect allows for fine tuning and tightening of the soft tissue to the bone during repair. The length of the suture connection **18** is variable and may be adjusted prior to selection of a tool or during surgical procedure through any appropriate means. Likewise, the diameter of the loop **22** is adjustable and may be replaced by one long strip of suture-like material, or molded strip, **26** having a slot or hole **28** for insertion of the spike or plug member once it has been passed through a tissue for attachment.

**FIG. 3** illustrates an alternate embodiment of the spike member with suturing material. The spike member **32** has attached at its rear end one complete loop of suture **34** which also has an adjustable diameter for various thicknesses of tissue reattachment.

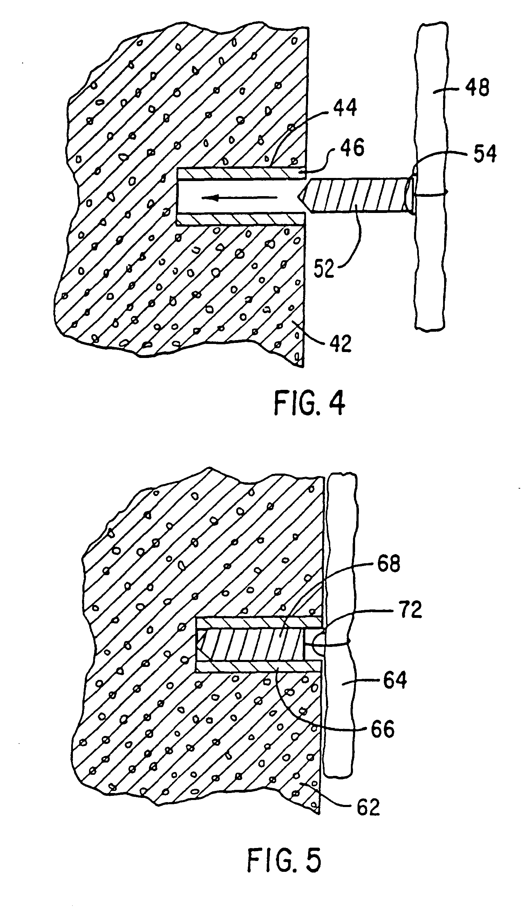

Referring now to **FIG. 4,** there is illustrated a process for reattachment of tissue to bone mass. Bone mass **42** has a slot or hole **44** wherein a hollow anchoring sleeve **46** has been inserted. A tissue **48** has inserted therein a spike member **52** threaded through loop **54** for reattachment to the bone mass **42.**

**FIG. 5** depicts the knotless suture anchor assembly in place after the surgical procedure has been completed. Bone mass **62** has tissue **64** attached or reattached thereto.

The surgical procedure for reattaching or attaching tissue to bone mass includes first installation of the hollow anchoring sleeve **66.** Secondly, the spike member **68** is inserted through the tissue **64** and then through at least one loop portion **72.** The spike **68** with tissue **64** securely attached thereto is then secured directly into the anchoring sleeve **66** for attachment of tissue **64** to bone mass **62** completing the method.

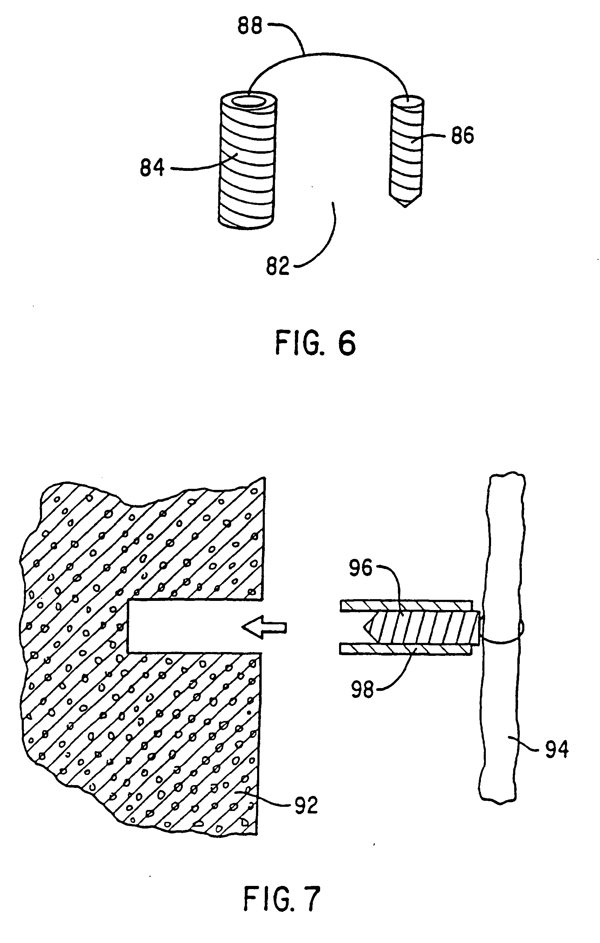

Referring now to **FIG. 6,** there is disclosed an embodiment of the knotless suture anchor system **82** to be used with the present invention. There is disclosed a hollow anchoring sleeve **84** having attached thereto a spike or plug member **86** by a suture element **88.** As discussed and described above in a preferred embodiment of the invention, the hollow anchoring sleeve **84** may take the shape or form of almost any type of anchor device. Configurations of the hollow anchoring sleeve having a collar, as well as a configuration incorporating any type of umbrella expansion means or threading on the exterior or interior of the sleeve are contemplated as being part of the present invention. Further, the spike or plug member **86** is preferably threaded on its exterior with a pointed end for insertion through a tissue element. This may take the form of any structure and preferably has an exterior which is ribbed, beaded, threaded, smooth or expandable. Likewise, the suture element **88** may be made of any acceptable surgical suture material and in addition is lengthened based upon thickness and need requirements.

Referring now to **FIG. 7,** there is depicted the initial step in the process for attachment of tissue to bone utilizing the alternate embodiment of **FIG. 6.** A spike member **96** is inserted through a tissue **94** and into a hollow anchoring sleeve **98.** The assembly is then attached to a bone mass **92.**

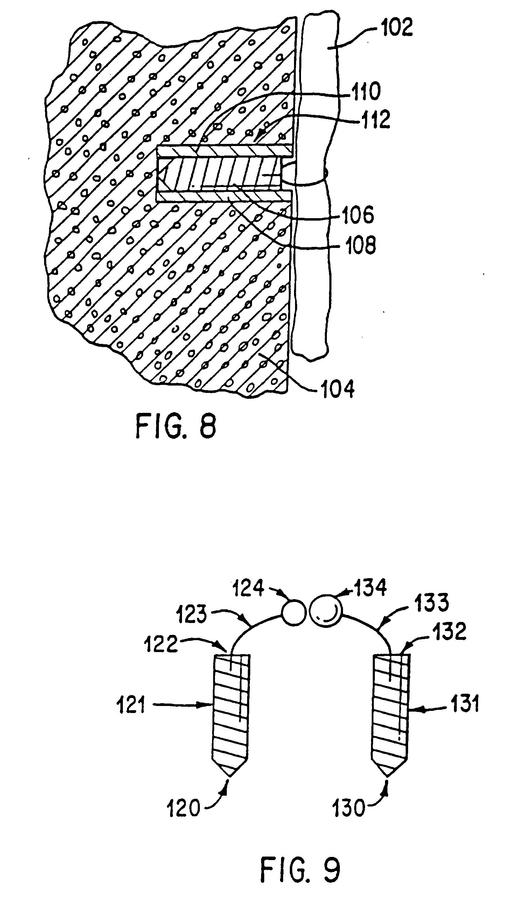

Referring now to **FIG. 8,** there is disclosed the completed attachment or reattachment of tissue **102** to bone mass **104.** The spike or plug member **106** is inserted through said tissue **102** and then inserted into hollow anchoring sleeve **108** for complete attachment to bone mass **104** at a previously drilled section **110.** This enables one to surgically reattach the tissue **102** to bone mass **104** in a virtual one step process.

Additionally, the process embodied by **FIG. 8** may be undertaken in two steps. First, the spike member **106** is inserted through the soft tissue **102** and into the hollow anchoring sleeve **108.**

Secondly, the entire assembly **112,** spike member **106** which has been inserted into the sleeve **108,** is inserted into the bone mass **104.** Regardless of the process used, the repair tightness is adjusted by the depth of anchor insertion into bone and/or the ratcheting effect of the spike in the anchor sleeve.

**FIG. 9** depicts a preferred embodiment of the present invention. First and second spike or plug assembly members, **120** and **130,** respectively, are depicted for the knotless suture anchor assembly. There is disclosed first spike or plug assembly member **120** having spike means **121** for insertion into a hollow anchoring sleeve as depicted in **FIGS. 1a - 1d.** Spike or plug member **121** is preferably threaded with a pointed end for insertion through a tissue element and secure fixation with said hollow anchoring sleeve. The spike or plug member may take the form of any structure and preferably has an exterior which is ribbed, threaded, beaded, expandable or smooth. The diameter of thickness of the member is preferably equal to or incrementally less than the entire diameter of the hollow anchoring sleeve for a secure fit. In situations where ribs, threads, beads, or the like, are utilized on the spike member, the diameter of the member with protrusions may initially exceed the inner diameters of the hollow anchoring sleeve and would deform upon insertion.

The rear end **122** of the spike member **121** has attached or tied thereto a suture element **123.** The suture element **123** has attached or molded therewith a catch device, such as a ring element, **124** which is preferably rigid. The spike member **121,** suture element **123** and catch device (ring element) **124** may be made of separate materials or molded together as one piece. In addiction, the spike member **121** and suture element **123** may be molded as one element or the suture element **123** and catch device (ring element) **124** may be molded as one element.

Second spike assembly member **130** includes a spike or plug member **131** for insertion into a hollow anchoring sleeve as depicted in **FIGS. 1a - 1d.** The element **131** is described above and can be the same or different as spike element **121.** To the rear end **132** of the spike member **131** there is tied or attached a suture element **133.** The suture element **133** has attached or molded therewith a spherical, or the like, stop means **134.**

As stated above, the three components, spike member **131,** suture element **133** and stop means **134** can be combined as separate components or molded together as one entire component or a combination of two components.

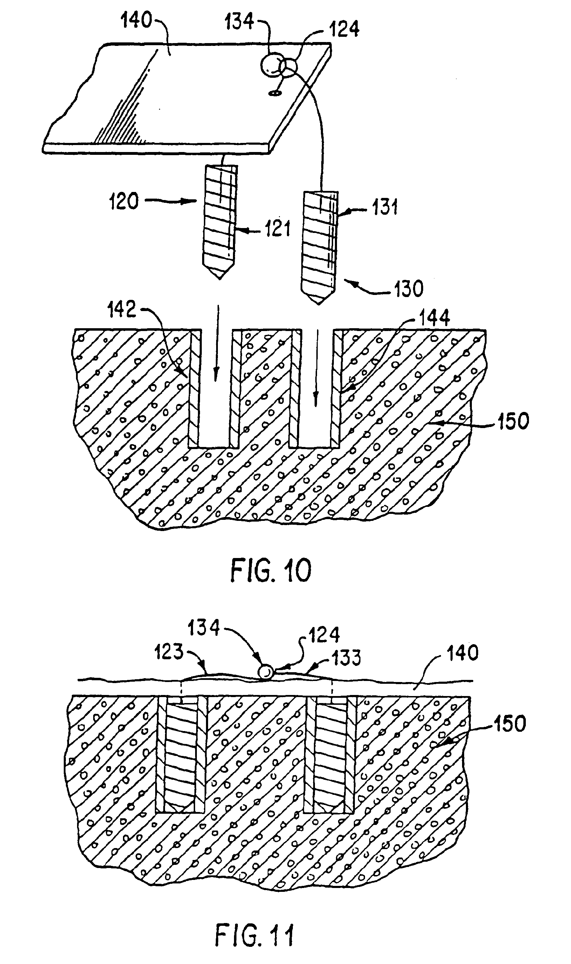

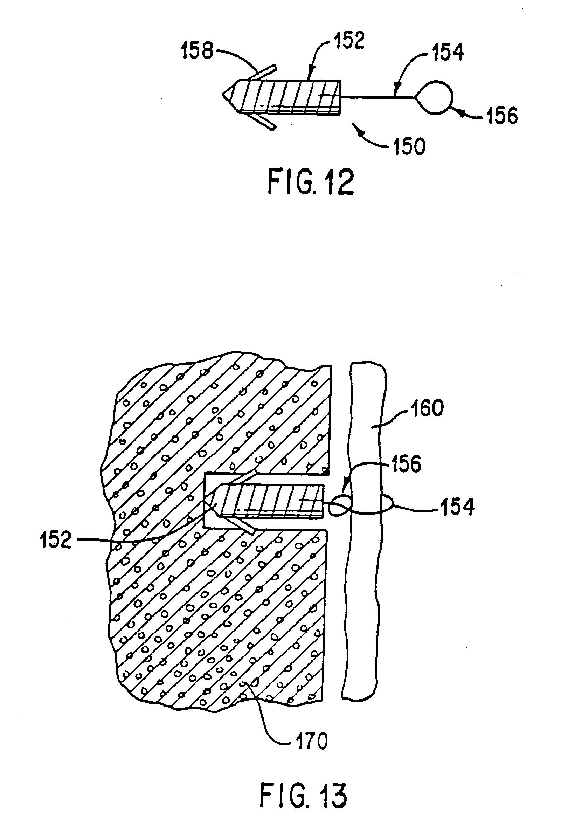

Referring now to **FIG. 10,** first spike assembly member **120** is inserted into tissue **140.** The spike member **121** can be inserted into a hollow anchoring sleeve **142** either before or after the hollow anchoring sleeve **142** is inserted into bone mass **150.** Spike member **131,** of spike member assembly means **130,** is inserted through catch device (ring element) **124,** of spike member assembly **120.** The spike member **131** is then inserted through tissue **140** and then into hollow anchoring sleeve **144,** or directly into hollow anchoring sleeve **144.** The hollow anchoring sleeve **144** can be anchored to bone mass **150** before or after spike member **131** has been inserted therein. The stop means **134** mates with catch device (ring element) **124** dragging the tissue into a secure fit with bone mass **150** (See **FIG. 11**.)

Once the spike members have been inserted into the hollow anchoring sleeves they can be ratcheted down to a desired depth to adjust the tightness of the soft tissue repair to bone mass. Tightness can also be adjusted by the depth of anchor sleeve insertion into the bone mass.

Stop means **134** may be spherical with a diameter greater than catch device (ring element) **124,** or may be any shape which would facilitate a mating with the catch device. Likewise, the catch device need not be circular, but requires only an opening to allow passage of a spike member and prevent passage of the stop means. The catch device **124,** depicted in **FIGS. 10** and 11, can take the form of a ring, square, slot, or any shape that will cooperate and hold stop means **134** from being pulled therethrough.

Knotless suture anchor assembly **150** is an alternate embodiment of the present invention (**FIG. 12**). The assembly **150** includes a spike/anchor means **152,** a suture element **154,** and a loop portion **156.** As discussed above, the suture element **154** and loop portion **156** may be made of the same or different materials. In addition, the spike/anchor means **152** can be molded with the suture element **154** and loop portion **156** as one piece or as separate components, as desired. The spike/anchor means can be configured, as desired, to securely attach the assembly **150** to a bone mass. The spike/anchor means may include all the attachment means as described above for the hollow anchoring sleeve such as harpoon type means **158,** prongs, umbrella spokes, threads, wedges or the like.

Additionally, the entire suture element **154** may be configured in a complete loop as depicted in **FIG. 3,** or may merely have a slit along its length as depicted in **FIG. 2.**

When performing a procedure, loop portion **156** is pulled through soft tissue **160.** Once the loop portion **156** has been pulled through the soft tissue **160,** the spike/anchor means **152** is inserted through the loop portion **156** and directly into bone mass **170** for attachment of tissue **160** to bone mass **170.** The length of suture **154** and depth of insertion of the spike/anchor means **152** controls the secure tightness of repair of tissue **160** to bone mass **170,** and is adjusted as desired.

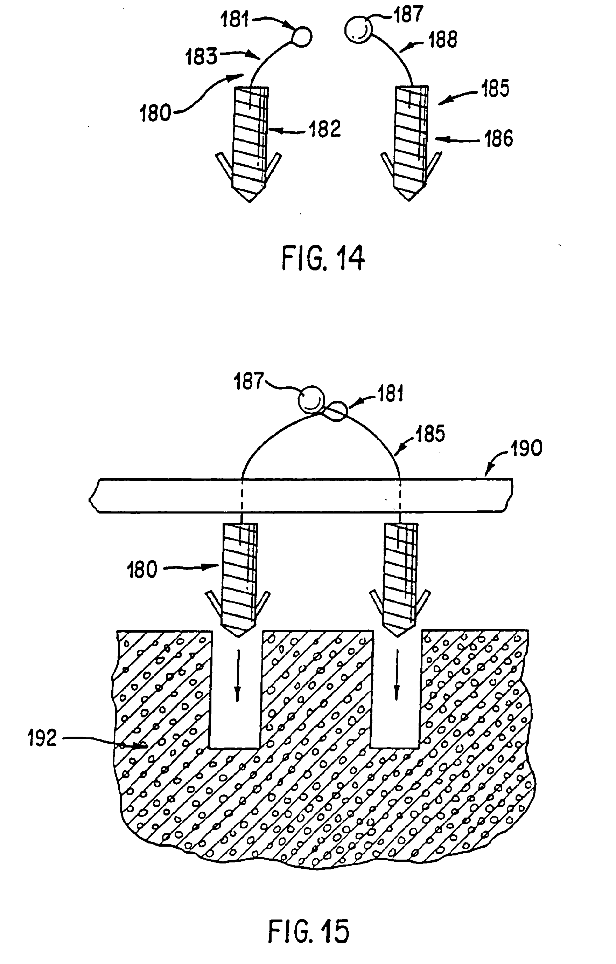

**FIG. 14** depicts an embodiment of the present invention wherein a first knotless suture anchor assembly **180** is provided as described in accordance with knotless suture anchor assembly **150,** as depicted in **FIG. 12** and previously described. A second knotless suture anchor assembly **185** is also provided to being used in conjunction with assembly **180** to perform a surgical attachment of tissue to bone.

First the catch device **181** is inserted through soft tissue **190** from the underside of the tissue first (see **FIG.** 15). Then a spike/anchor means **182** of assembly **180** is inserted into the bone mass **192.** Next, a spike/anchor means **186** (with bone anchoring features) is threaded through catch device **181** and inserted into bone mass **192.** Stop means **187** mates with catch device **181** and the sutures **183** and **188** are pulled taught to securely attach soft tissue **190** to bone mass **192.**

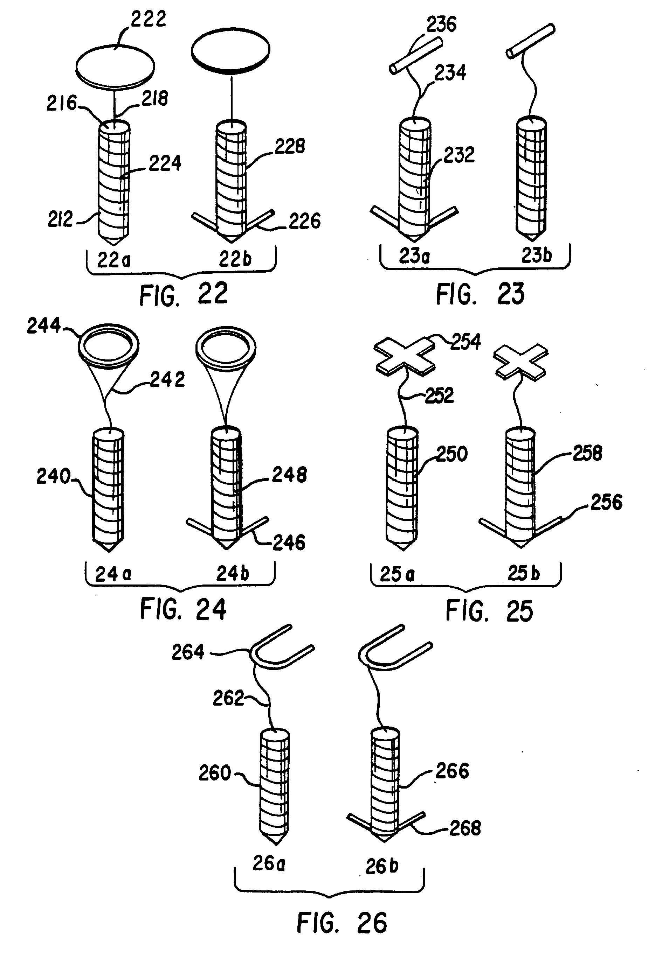

**FIG. 22,** including **22a** and **22b,** shows a perspective view of the spike plug member with suture element and stop means, of the two-piece and one-piece embodiment, respectively, to be used with the present invention. Spike or plug member **212** is preferably cylindrical in shape with a sharp first end **214** and a second end **216** wherein the suture element **218** is attached. The suture element **218** has at its distal end a disc-like stop means **222.** The stop means **222** can be constructed of any material may be one molded component and attached to spike member **212** at end **216.** Further, the suture element **218,** alone, can be made from any type suture material which has been approved for surgical procedures or a molded material for attachment of tissue to bone. The spike or plug member can form any shape so long as it mates with the hollow cylindrical sleeve as described above. The exterior of the spike or plug member **212** may be ribbed or threaded **224** as depicted in **FIG. 22** or may be beaded or expandable to allow for a secure tight fit with the inner hollow cylinder of the anchoring sleeve. Once inserted into a hollow anchoring sleeve, the exterior surface of the spike or plug member **212** engages the inner surface of the sleeve and can be ratcheted down to produce the desired tight fit. The interface of the spike and sleeve allows for movement of the spike in only one direction and resists pullout or movement out of the sleeve. This ratcheting effect allows for fine tuning and tightening of the soft tissue to the bone during repair. The length of the suture connection **218** is variable and may be adjusted prior to selection of a tool or during surgical procedure through any appropriate means. Likewise, the diameter of the disc-like stop means **222** is adjustable. **FIG. 22b** is identical to **22a** but for anchoring means **226** attached to the spike or plug member **228.** This one-piece embodiment allows for tissue reattachment without an anchoring sleeve. The spike or plug member anchors directly into the bone mass.

**FIG. 23** illustrates an alternate embodiment of the spike member with suturing material and stop means. In **FIG. 23a,** the spike member **232** has attached at its rear a suture **234** and rod-like stop means **236.** This is the embodiment of the one-piece anchor. **FIG. 23b** illustrates the spike or plug member of the two-piece embodiment when used in combination with an anchoring sleeve.

**FIG. 24** illustrates an alternate embodiment of the spike member with suturing material and stop means. In **FIG. 24a,** the two-piece embodiment, Spike member **240** has attached thereto at least one suture means **242** connected to a stop means **244** configured in the shape of a ring or hoop. **FIG. 24b** illustrates the one-piece embodiment wherein anchoring means **246** is attached to the spike or plug member **248** for attachment to bone without an anchoring sleeve and hence not falling under the scope of claim 1 according to the invention.

**FIG. 25** contains alternate embodiments **25a** and **25b. FIG. 25a,** the two-piece embodiment, includes a spike means **250,** a suture means **252** attached thereto, and an X-like stop means **254. FIG. 25b,** the one-piece embodiment, illustrates an embodiment of the configuration wherein anchoring means **256** is attached to the spike or plug means **258** for attachment to bone when an anchoring sleeve is not utilized.

**FIG. 26** contains **FIGS. 26a** and **26b** which are alternate embodiments of the invention including a spike member **260,** suturing means **262** attached thereto, and a stop means **264** configured in a horseshoe configuration. **FIG. 26b** illustrates an alternate embodiment of the horseshoe configuration wherein the spike or plug member **266** has anchoring means **268** attached thereon for direct attachment to bone without the utilization of an anchoring sleeve.

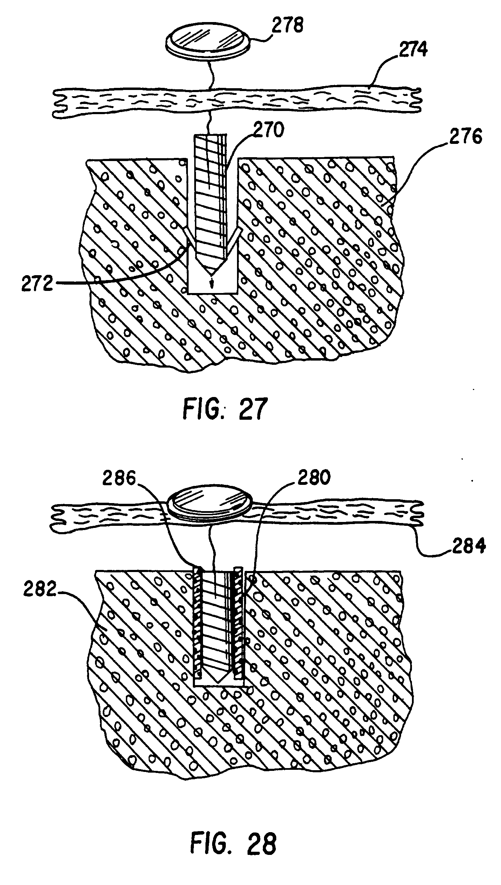

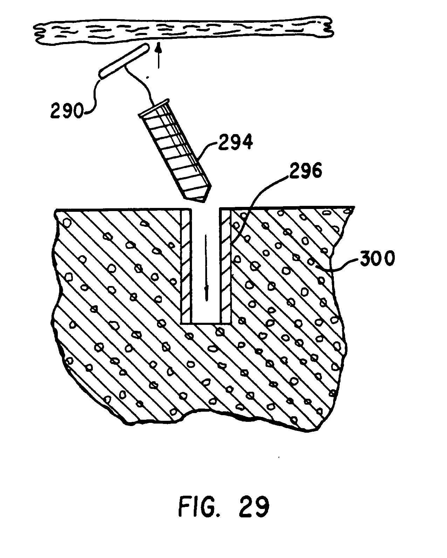

Referring now to **FIG. 27** and **FIG. 28,** there is illustrated a surgical procedure for reattaching or attaching tissue to bone depicting the spike or plug embodiments illustrated in **FIG. 22.** The procedure can be enacted for any of the embodiments outlined in **FIGS. 22, 23, 24, 25** and **26. FIG. 27** illustrates the procedure wherein an anchoring sleeve is not utilized. Spike or plug means **270** having anchoring means **272** is inserted through tissue **274** and directly into bone **276.** The stop means **278** grabs the tissue **274** and pulls same back into reattachment or attachment with bone **276** when the spike or plug member is forced into the opening in the bone. The tightness of the repair is adjusted by the length of suture **270** and/or the depth of the insertion of spike member **272** into the bone mass.

**FIG. 28** depicts a procedure wherein an anchoring sleeve **280** is first inserted into bone mass **282.** Subsequent to the insertion of the anchoring sleeve **280,** a spike or plug member **286** is inserted through tissue **284** and into the anchoring sleeve **280.** The spike or plug member **286** is then ratcheted down into the anchoring sleeve **280** to pull tissue mass **284** into direct and secure mating with bone mass **282.**

Referring now to **FIG. 29,** there is an alternate surgical procedure disclosed for utilization of the rod-like stop means depicted in **FIG. 23.** Initially the rod-like stop means 290 is inserted through tissue mass **292.** Once the rod-like stop means rests on top of the tissue mass, the spike or plug member **294** is then inserted into a previously inserted anchoring sleeve **296.** The spike or plug member **294** is then ratcheted down into the anchoring sleeve for secure mating or attachment of the tissue **292** to the bone mass **300.** This procedure may also be undertaken with the one-piece anchor having a spike or plug means as depicted in **FIG. 23a** which omits the initial insertion of an anchoring sleeve.

In addition to the shapes illustrated for the stop or catch means portion of the invention, the stop means can be any planar or non-planar shape such as, but not limited to, C-shaped, planar with one or more openings, bar-shaped, curved or non-planar bar-shaped. Further the stop means is attached to the spike or plug member by one or more suture elements. The suture element or connection can be made up of a known suture material such as Ethibond® or Prolene®, or it can be made of polymer materials such as ultrahigh molecular weight polyethylene. The connection or suture element can be formed of bio-absorbable material such as a polylactide polymer. Additionally, the suture element can be part of the stop means and formed by a molding process or the like.

The suture element can be connected to the stop means and an anchor in a variety of ways such as fusion or molding or by mechanical means such as glue, a weld or by mere tieing.

In many situations throughout the discussion above, the terminology secure attachment of soft tissue to bone has been used. Such terminology refers to the attachment or reattachment of tissue to bone through the insertion of a spike member into a hollow anchoring sleeve or a spike/anchor means into a bone mass. In the former situation, the spike member can seat into the sleeve in a one step mating procedure or be inserted and ratcheted down in a step wise fashion into the sleeve. Either situation will function effectively and selection is based upon the instant facts of the surgical procedure. Further, the sleeve itself may be seated in the bone mass at varying depths. Again, such depth is a selection based upon the facts of the instant procedure. In the latter situation, where a spike/anchor means is used, depth of insertion of the device into the bone is a selection or choice of the surgeon during the procedure. In all situations, the spike member or spike/anchor means is designed not to back up or exit once mated with the sleeve, ratcheted down into the sleeve, or inserted into the bone mass to avoid and prevent withdrawal therefrom.

The spike or plug member can be made for direct insertion into the bone with screws, prongs, spikes, a wedge means or any means wherein the spike or plug member anchors securely into the bone mass facilitating attachment or reattachment of tissue to skin.

Further, the spike or plug member or a portion of the spike or plug member may be made with bioabsorbable material.

While a preferred embodiment of the invention in a knotless suture anchor system has been shown and described herein, it should be understood that the present disclosure is made by way of example only and that variations to the structure shown and its use are possible within the scope of this disclosure without departing from the subject matter coming within the scope of the following claims, and a reasonable equivalency thereof, which claims we regard as our invention.

## Claims

1. A knotless suture anchor assembly comprising:
a) a first anchor means assembly (120) comprising a first anchor means (121) having a first end (122) and a second end, said first end (122) of said first anchor means (121) having a suture element (123) attached thereto, wherein said suture element (123) has a catch device (124) located along its length;
b) a second anchor means assembly (130) comprising a second anchor means (131) having a first end (132) and a second end, said first end (132) of said second anchor means (131) having a suture element (133) attached thereto, wherein said suture element (133) has a stop means (134) located along its length for engagement with said catch device (124), wherein said second anchor means (131) is passed through said catch device (124) of said first anchor means assembly (120) until said stop means (134) is engaged with said catch device (124) and said second anchor means (131) is thereby engaged with said first anchor means (121); and
c) two hollow anchoring sleeves (142, 144) for installation and anchoring to a bone mass for receiving said first and second anchor means (121, 131) after said second anchor means (131) has been passed through said catch device (124) of said first anchor means (121).

2. A knotless suture anchor assembly as claimed in Claim 1, wherein said first and second anchor means (121, 131) have exterior surfaces which are smooth, ribbed, threaded, beaded or expandable, for secure engagement of said anchor means (121, 131) with said hollow anchoring sleeves (142, 144).

3. A knotless suture anchor assembly as claimed in Claim 1, wherein said hollow anchoring sleeves (142, 144) have exterior surfaces which are ribbed, threaded, pronged, or smooth for secure engagement of said sleeves (142, 144) with said bone mass.

4. A knotless suture anchor assembly as claimed in Claim 1, wherein said two hollow anchoring sleeves (142, 144) have interior surfaces which are ribbed, threaded, or smooth for secure engagement of said anchor means (121, 131).

5. A knotless suture anchor assembly as claimed in Claim 1, wherein said two hollow anchoring sleeves (142, 144) have a first end and a second end and a collar attached to said first end of flush engagement of said hollow anchoring sleeve (142, 144) with said bone mass.

## Patentansprüche

1. Knotenlose Nahtankeranordnung, umfassend:
a) eine erste Ankermittelanordnung (120) umfassend ein erstes Ankermittel (121), das ein erstes Ende (122) und ein zweites Ende aufweist, wobei das erste Ende (122) des ersten Ankermittels (121) ein daran befestigtes Nahtelement (123) aufweist, wobei das Nahtelement (123) eine auf seiner Länge angeordnete Riegelvorrichtung (124) aufweist;
b) eine zweite Ankermittelanordnung (130) umfassend ein zweites Ankermittel (131), das ein erstes Ende (132) und ein zweites Ende aufweist, wobei das erste Ende (132) des zweiten Ankermittels (131) ein daran befestigtes Nahtelement (133) aufweist, wobei das Nahtelement (133) ein auf seiner Länge angeordnetes Anschlagmittel (134) zum Eingriff in die Riegelvorrichtung (124) aufweist, wobei das zweite Ankermittel (131) durch die Riegelvorrichtung (124) der ersten Ankermittelanordnung (120) geführt wird, bis das Anschlagmittel (134) in die Riegelvorrichtung (124) eingreift und das zweite Ankermittel (131) **dadurch** in das erste Ankermittel (121) eingreift; und
c) zwei hohle Verankerungshülsen (142, 144) zum Anbringen und Verankern an einer Knochenmasse zur Aufnahme der ersten und zweiten Ankermittel (121, 131), nachdem das zweite Ankermittel (131) durch die Riegelvorrichtung (124) des ersten Ankermittels (121) geführt worden ist.

2. Knotenlose Nahtankeranordnung nach Anspruch 1, bei der die ersten und zweiten Ankermittel (121, 131) glatte, gerippte, mit Gewinde versehene, gesickte oder dehnbare Außenflächen zum sicheren Eingriff der Ankermittel (121, 131) in die hohlen Verankerungshülsen (142, 144) aufweisen.

3. Knotenlose Nahtankeranordnung nach Anspruch 1, bei der die hohlen Verankerungshülsen (142, 144) gerippte, mit Gewinde versehene, gezinkte oder glatte Außenflächen zum sicheren Eingriff der Hülsen (142, 144) mit der Knochenmasse aufweisen.

4. Knotenlose Nahtankeranordnung nach Anspruch 1, bei der die beiden hohlen Verankerungshülsen (142, 144) gerippte, mit Gewinde versehene oder glatte Innenflächen zum sicheren Eingriff der Ankermittel (121, 131) aufweisen.

5. Knotenlose Nahtankeranordnung nach Anspruch 1, bei der die beiden hohlen Verankerungshülsen (142, 144) ein erstes Ende und ein zweites Ende und einen am ersten Ende befestigten Kragen zum bündigen Eingriff der hohlen Verankerungshülse (142, 144) mit der Knochenmasse aufweisen.

## Revendications

1. Ensemble d'ancrage de suture sans noeud comprenant :
a) un premier ensemble de moyens d'ancrage (120) comprenant un premier moyen d'ancrage (121) ayant une première extrémité (122) et une seconde extrémité, ladite première extrémité (122) dudit premier moyen d'ancrage (121) ayant un élément de suture (123) attaché à celui-ci, dans lequel ledit élément de suture (123) possède un dispositif d'arrêt (124) situé sur sa longueur ;
b) un second ensemble de moyens d'ancrage (130) comprenant un second moyen d'ancrage (131) ayant une première extrémité (132) et une seconde extrémité, ladite première extrémité (132) dudit second moyen d'ancrage (131) ayant un élément de suture (133) attaché à celui-ci, dans lequel ledit élément de suture (133) possède un moyen d'arrêt (134) situé sur sa longueur pour s'engager avec ledit dispositif d'arrêt (124), dans lequel ledit second moyen d'ancrage (131) est passé au travers dudit dispositif d'arrêt (124) dudit premier ensemble de moyens d'ancrage (120) jusqu'à ce que ledit moyen d'arrêt (134) soit engagé avec ledit dispositif d'arrêt (124) et ledit second moyen d'ancrage (131) est ainsi engagé avec ledit premier moyen d'ancrage (121) ; et
c) deux manchons creux d'ancrage (142, 144) pour une installation et un ancrage à une masse osseuse pour recevoir lesdits premier et second moyens d'ancrage (121, 131) après que ledit second moyen d'ancrage (131) a été passé au travers dudit dispositif d'arrêt (124) dudit premier moyen d'arrêt (121).

2. Ensemble d'ancrage de suture sans noeud selon la revendication 1, dans lequel lesdits premier et second moyens d'ancrage (121, 131) présentent des surfaces extérieures lisses, striées, filetées, perlées ou extensibles, pour un engagement sûr desdits moyens d'ancrage (121, 131) avec lesdits manchons creux d'ancrage (142, 144).

3. Ensemble d'ancrage de suture sans noeud selon la revendication 1, dans lequel lesdits manchons creux d'ancrage (142, 144) présentent des surfaces extérieures striées, filetées, en pointes ou lisses pour un engagement sûr desdits manchons (142, 144) avec ladite masse osseuse.

4. Ensemble d'ancrage de suture sans noeud selon la revendication 1, dans lequel lesdits deux manchons creux d'ancrage (142, 144) présentent des surfaces intérieures striées, filetées ou lisses pour un engagement sûr desdits manchons (121, 131).

5. Ensemble d'ancrage de suture sans noeud selon la revendication 1, dans lequel lesdits deux manchons creux d'ancrage (142, 144) présentent une première extrémité et une seconde extrémité et un collet attaché à ladite première extrémité à engagement affleurant dudit manchon creux d'ancrage (142, 144) avec ladite masse osseuse.
